# EUROPEAN PATENT SPECIFICATION

(11) **EP 3 000 475 B1**
(45) Date of publication and mention of the grant of the patent: **22.05.2019**
(21) Application number: 14801136.4
(22) Date of filing: 10.04.2014
(51) Int. Cl.: A61K 38/49, A61P 17/02, A61K 38/48

(54) **PHARMACEUTICAL OR COSMETIC COMPOSITION FOR THE REGENERATION AND CICATRISATION OF THE SKIN**
PHARMAZEUTISCHE ODER KOSMETISCHE ZUSAMMENSETZUNG ZUR REGENERIERUNG UND VERNARBUNG DER HAUT
COMPOSITION PHARMACEUTIQUE OU COSMÉTIQUE POUR LA RÉGÉNÉRATION ET LA CICATRISATION DE LA PEAU

(30) Priority: 23.05.2013 ES 201330747
(43) Date of publication of application: 30.03.2016
(73) Proprietor: Kledos Cell Tech, S.L., 08213 Barcelona (ES)
(72) Inventor: GALEANO SALVADOR, José Manuel, E-08213 Barcelona (ES); FERNÁNDEZ PIN, José Manuel, E-08213 Barcelona (ES); LABRADOR BARRAFÓN, Daniel, E-08213 Barcelona (ES); KLEIN, Georges R., E-08213 Barcelona (ES); GONZÁLEZ AZUARA, Nuria, E-08213 Barcelona (ES)
(74) Representative: Carpintero Lopez, Francisco
(86) International application number: PCT/ES2014/070298
(87) International publication number: WO 2014/188027

(56) References cited:
- EP-A1- 0 723 782
- WO-A1-93/25209
- WO-A1-03/084410
- GB-A- 2 409 159
- US-A1- 2003 133 960
- US-A1- 2003 147 876
- US-A1- 2007 003 502
- Anonymous: "bT-Infusion oxygen masque", , 30 November 2012 (2012-11-30), pages 1-1, XP055322910, Retrieved from the Internet: URL:http://www.bio-therapeutic.co.uk/bT-C_ PDF/OXYGENMASQUE4-10-12.pdf [retrieved on 2016-11-24]
- VERALDI S ET AL: "Treatment of pruritus in mild-to-moderate atopic dermatitis with a topical non-steroidal agent", JOURNAL OF DRUGS IN DERMATOLOGY, STRATEGIC COMMUNICATION IN DERMATOLOGY, NEW YORK, NY, US Journal of Drugs in Dermatology, vol. 8, no. 6 1 June 2009 (2009-06-01), pages 537-539, XP008170338, ISSN: 1545-9616 Retrieved from the Internet: URL:http://jddonline.com/articles/dermatol ogy/S1545961609P0537X/1
- CÉLINE COUTEAU ET AL: "Influence of certain ingredients on the SPF determined in vivo", ARCHIVES OF DERMATOLOGICAL RESEARCH ; FOUNDED IN 1869 AS ARCHIV FÜR DERMATOLOGIE UND SYPHILIS, SPRINGER, BERLIN, DE, vol. 304, no. 10, 16 June 2012 (2012-06-16), pages 817-821, XP035143977, ISSN: 1432-069X, DOI: 10.1007/S00403-012-1257-X
- THOMAS H BUGGE ET AL: "Urokinase-type plasminogen activator is effective in fibrin clearance in the absence of its receptor or tissue-type plasminogen activator", PROCEEDINGS OF THE NATIONAL ACADEMY OF SCIENCES, vol. 93, no. 12, 11 June 1996 (1996-06-11) , pages 5899-5904, XP055323249,
- YUE SHEN ET AL: "Mice Deficient in Urokinase-Type Plasminogen Activator Have Delayed Healing of Tympanic Membrane Perforations", PLOS ONE, vol. 7, no. 12, 7 December 2012 (2012-12-07), page e51303, XP055323256, DOI: 10.1371/journal.pone.0051303
- P. A. JIMENEZ ET AL: "Urokinase-type plasminogen activator stimulates wound healing in the diabetic mouse", INFLAMMATION RESEARCH, vol. 46, no. 0, 3 December 1997 (1997-12-03), pages 169-170, XP055323881, CH ISSN: 1023-3830, DOI: 10.1007/s000110050164
- DATABASE WPI Week 199524, Derwent Publications Ltd., London, GB; Class B04, AN 1995-181632, XP055295751 'SKIN EXTERNAL AGENT, HAVING HIGH WOUND-CURING EFFECTS - CONTAINS SINGLE-CHAIN UROKINASE TYPE PLASMINOGEN ACTIVATOR.' & JP H06 321 806 A (SHISEIDO CO LTD) 22 November 1994

## Description

### OBJECT OF THE INVENTION

The present invention falls within the field of the pharmaceutical and cosmetic industry. It specifically refers to a pharmaceutical or cosmetic composition comprising urokinase enzyme, in combination with allantoin and bisabolol as active ingredients. In addition this pharmaceutical or cosmetic composition comprises at least one active ingredient selected from active ingredients with regenerating function, such as hyaluronic acid, collagen, rosehip oil, boswellia serrata extract, antioxidants, enzymes and growth factors.

The present invention also relates to a dosage form for the topical administration of a pharmaceutical or cosmetic composition comprisingurokinase enzyme, allantoin and bisabololas active ingredients. Additionally, the dosage form comprises, at least, one active ingredient selected from active ingredients with regenerating function, such as hyaluronic acid, collagen, rose hip oil, boswellia serrata extract, antioxidants, enzymes and growth factors.

The present invention also relates to uses of said pharmaceutical or cosmetic composition and the dosage form for topical application, particularly for the healing and regeneration of significant skin disorders, in order to obtain a normotrophic evolution of these disorders.

### STATE OF THE ART

Regeneration and healing of the skin are two critical processes which ensure the proper functioning of this organ and a favourable aesthetic of the same. Because of multiple genetic and environmental factors, these processes do not always happen within the correct time period and correct way, therefore external elements are needed to ensure the success of both.

During the regeneration process a restitution of damaged or lost tissue is made, consisting of the growth of cells and other structures that replace those affected. The skin is one of the few tissues-organs where there is a higher rate of regeneration, provided that the stem cells that give rise to new tissues have not been destroyed.

For the healing processes of the skin, these involve restoring the original structures with deposits of collagen. Basically this occurs in three major phases, which are: the inflammatory phase, the proliferative phase and the remodelling or maturation phase. The last phase or remodelling phase can last months or years and is characterised by a remodelling of the deposited collagen.

As to the evolution that the skin healing may have, a normotrophic scarring or evolution is desired that occurs when the skin acquires the same or very similar textural aspect and consistency to that presented before the injury or trauma.

As indicated above, in order to ensure the successful development of these processes, usually external elements, such as moisturising solutions, creams, etc., based on natural or synthetic substances involved in the regeneration and healing processes are used, as they improve the final appearance of scars already established.

Treatments available today are very varied, being specific to each of the healing phases. There are specific dressings that guarantee an environment and the physical chemical conditions of pH, temperature and humidity ideal for promoting the healing process. Moreover, by technically knowing each of the healing phases, treatments and techniques have appeared, which are appropriate to each of these stages. For example, if the wound to be treated presents devitalized tissue or slough, either surgical techniques or enzymatic techniques are used. To favour the development of a proliferative or granulation tissue other types of compounds are used. Finally other preparations are involved in the final epithelialisation and remodelling to prevent the formation of unsightly keloids.

If there is discomfort, or aches and pain during this process, analgesics or anti-inflammatory agents are used to improve the patient's well-being.
For this reason, sometimes, for the treatment of a wound, a huge therapeutic arsenal is used due to the lack of a single integrative treatment.

Among the compounds known and commonly used is allantoin (glyoxyl-diurea), which has epithelialising action and produces increased moisturising, which promotes wound healing. Allantoin is the end product of purine metabolism, found in animal and plant organisms. Its use on scars was known, even before its benefits were scientifically demonstrated. It has keratolytic properties, favourable for penetration and anti-inflammatory effects, and reduces the frequent itching of the healing process.

In the case of bisabolol, an essential oil extracted from the chamomile flower, it can be a constituent of healing, soothing and/or anti-inflammatory creams.

Less well known in the therapeutic or cosmetic preparations for the treatment of skin regeneration and healing is the urokinase enzyme.
This enzyme is originally obtained from human urine. It has a recognised thrombolytic effect by activating plasminogen to plasmin, which hydrolyzes fibrin networks, thus preventing the clogging of blood vessels, arterial-venous problems and pleural effusions, so its only known routes of administration are endovascular, endo-catheter and intra-pleural infusion.

That is why urokinase is used as lyophilized powder dissolved in isotonic saline, and the usual form of administration is by continuous intra-venous perfusion. Under these conditions the use of the same is restricted to the hospital environment.

An example of using urokinase as a thrombolytic agent is disclosed in patent application WO9823151, which describes a method for preventing vascular diseases by administering low doses of plasminogen activators such as urokinase, for long periods of time.

In the patent application WO9515747 the use of fibrinolysis enhancing agents contained in biocompatible and biodegradable matrices is disclosed specifically for the treatment of surgical adhesions.

Taking the state of the art into consideration, currently a pharmaceutical or cosmetic preparation useful as a comprehensive treatment and which is effective and efficient in terms of time reduction in the skin regeneration and healing process is desirable, particularly for the treatment of significant skin disorders, achieving satisfactory results in terms of the aesthetic appearance of the area of skin that has been damaged, to the same or very similar condition to that it had before suffering the disorder.

Consequently from the above, a need exists for a dosage form that allows the easy application of the aforementioned pharmaceutical or cosmetic preparation and that ensures the effectiveness and stability of the active ingredients. Furthermore, a simplification in the therapeutic and/or cosmetic treatments related to the regeneration and healing of significant skin disorders is desirable, this simplification aimed specifically at reducing the medical supplies to be used and reducing the time used in these processes of regeneration and healing.

### DESCRIPTION OF THE INVENTION

The inventors of the present invention have surprisingly discovered that urokinase induces rapid clot dissolution and the liquefaction of fibrinous exudates, exerting a beneficial response to stimulate and promote the healing process and healing of surgical wounds, infected or not, and other significant changes in the skin, improving the cleaning of necrotic tissue.

For purposes of the present invention, significant skin disorders means any disruption or discontinuity in the epidermis and even the dermis. This includes from superficial erosions and minor irritations to ulcer type injuries of venous or arterial origin, pressure ulcers, diabetic foot, traumatic wounds and wounds of surgical origin.

Also, within the scope of this invention, the fact that the regeneration and healing of skin involves phases which overlap and are extended or shortened in time, depending on various factors, is considered. Generally four main phases can be considered, which are:
- Inflammatory phase: a homoeostatic action occurs, in which platelets released by the damaged vessels come into contact with the disorganised collagen and simultaneously form a network thanks to the fibrin and fibronectin, causing the homoeostatic plug. Platelets release growth factors and stimulate fibrin production consolidating this network which will allow the migration of inflammatory cells to the injury site. Simultaneously, cells capable of producing substances with bactericidal action will arrive and other cells will release proteases that digest part of this network and help clean the wound.
- Granulation phase: cells called macrophages begin producing newly formed tissue and due to the reduction of oxygen in the area, the production of chemical factors will be stimulated that induce the formation of new blood vessels and thus, as if it were a sequence, granulation tissue and a new extracellular matrix will be produced. The presence of new blood vessels during this phase will give the wound an erythematous appearance due to increased blood, nutrients and water content. Besides, a specific cell type, fibroblasts, which are responsible for the production of collagen and extracellular matrix as supporting tissue, will arrive.
- Epithelialisation phase: during this phase the epithelial cells migrate across the new tissue already formed and start covering the wound. Basal keratinocytes from the wound margins will start covering the wound of epithelial tissue centripetally, needing for this constant relative humidity conditions.
- Skin remodelling phase: the wound is already epithelialised but the healing phase continues. There is a balance between the production and digestion of collagen deposited in the wound and the collagen is organised and structured following the lines of tension. The control of the balance between collagen production-degradation is carried out by proteins called metalloproteinases.

The present invention responds to all the above, disclosing a pharmaceutical or cosmetic composition comprising urokinase enzyme, allantoin and bisabolol as active ingredients. In addition, the pharmaceutical or cosmetic composition comprises, at least, one active ingredient selected from hyaluronic acid, collagen, rosehip oil, bosweilla serrata extract, antioxidants, enzymes and growth factors. Said composition quickly and efficiently exerts its regenerating and healing action on significant skin disorders and simplifies the therapeutic and/or cosmetic treatments related to the regeneration and healing of these disorders.

Another object of the present invention discloses a pharmaceutical or cosmetic composition comprising urokinase enzyme as active ingredient in a concentration of between 1 IU/ml and 10 OOOIU/ml.

Another object of the present invention is the use of the pharmaceutical or cosmetic composition comprising urokinase as active ingredient for the manufacture of a medicament or cosmetic for regeneration and healing of skin, in the granulation, epithelialisation and skin remodelling phases.

Another object of the present invention is the use of the pharmaceutical or cosmetic composition comprising urokinase in combination with allantoin and bisabolol as active ingredients and further comprising, at least, one active ingredient selected from regenerating active ingredients, such as hyaluronic acid, collagen, rosehip oil, boswella serrata extract, antioxidants, enzymes and growth factors for the preparation of a medication or cosmetic for the regeneration and healing of the skin.

Another object of the present invention is the use of the pharmaceutical or cosmetic composition comprising urokinase in combination with allantoin and bisabolol as active ingredients to be administered topically.

Another object of the present invention is the use of the pharmaceutical or cosmetic composition comprising urokinase in combination with allantoin and bisabolol as active ingredients and further comprising at least one active ingredient selected from active ingredients with regenerating function, such as hyaluronic acid, collagen, rosehip oil, boswellia serrata extract, antioxidants, enzymes and growth factors, for the preparation of a medication or cosmetic for regeneration and healing of skin, to be administered topically and in gel form.

Another object of the present invention is the use of pharmaceutical or cosmetic composition comprising urokinase in combination with allantoin and bisabolol as active ingredients and further comprising at least one active ingredient selected from active ingredients with regenerating function, such as hyaluronic acid, collagen, rosehip oil, boswellia serrata extract, antioxidants, enzymes and growth factors for the preparation of a medication or cosmetic for regeneration and healing of the skin, to be administered in a single daily dose over a period of 3 to 15 days.

Another object of the present invention is a dosage form for topical administration of an active ingredient, which is the urokinase enzyme in combination with allantoin and bisabolol, to be applied in gel form.

Another object of the present invention is a dosage form for topical administration of the urokinase enzyme in combination with allantoin and bisabolol and in association with at least one active ingredient selected from other active ingredients with regenerating function, such as hyaluronic acid, collagen, rosehip oil, boswellia serrata extract, antioxidants, enzymes and growth factors.

Another object of the present invention is a dosage form in gel form for topical administration of at least urokinase in combination with allantoin and bisabolol as active ingredients, and the dosage form further comprises at least one other active ingredient selected from active ingredients with regenerating function, such as hyaluronic acid, collagen, rosehip oil, boswellia serrata extract, antioxidants, enzymes and growth factors. The dosage form of the present invention in gel form maintains the effectiveness and stability of the urokinase enzyme for time periods of at least 15 days.

Another object of the present invention is a dosage form for topical administration, in an administration volume of less than 5 ml per application.

Another object of the present invention is the use of the dosage form for the manufacture of a medicament or cosmetic for treating significant skin disorders.

The pharmaceutical or cosmetic composition and the dosage form for topical administration in gel form, object of the present invention, comprises compounds selected from:

| Components: | Concentration (% w/w) |
|---|---|
| Urokinase (1 IU/ml- 10 000 IU/ml) | 0.01-1% |
| Regenerating active ingredients (e.g. allantoin, hyaluronic acid, collagen, rosehip oil) | 5-8 |
| Soothing active ingredients (e.g. bisabolol, boswellia serrata extract) | 0.01-7 |
| Emollients (e.g. vegetable oils, isopropyl myristate, fatty alcohols) | 1-25 |
| Humectants (e.g. glycerine, propylene glycol) | 1-25 |
| Viscosity regulating agents (e.g. cellulose ethers, carbomer, gum arabic, guar gum, xanthan gum) | 0.5-8 |
| Preservatives (antimicrobial and antifungal) | 0.01-8 |
| pH regulators (acids and bases) | 0.1-10 |
| Chelating agents (e.g. EDTA) | 0.05-5 |
| Other active ingredients (antioxidants, e.g. tocopherol), (biological extracts, e.g. enzymes, growth factors), (botanical extracts) | 0.01-3 |
| Water | q.s. 100 |

The concentrations being % w/w, such that the individual values of the different components of the composition are such that the total of the composition never exceeds 100%, and wherein the gel maintains the effectiveness and stability of the enzyme for time periods of at least 18 months in an unopened container and 3 months once the container has been opened.

After a long experience in the treatment of scars and other significant skin disorders, it has surprisingly been found that urokinase induces rapid clot dissolution and liquefaction of fibrinous exudates in these skin disorders, by stimulating and promoting the regeneration and healing process thereof, whether they are infected or not.

Specifically, it has been found that the action of urokinase consists in degrading the fibrin networks and slough that are produced under pathological conditions and that prevent the correct regeneration and healing of wounds, ulcers and other significant skin disorders.

During the healing phase, the action of urokinase is to stimulate and activate angiogenic factors which induce the formation of new blood vessels, which will involve a greater and better supply of oxygen and nutrients to the wound bed of the skin disorder. Simultaneously, it will eliminate the presence of fibrin, promoting the granulation phase.

Finally a cosmetic remodelling of the scar will occur removing the excess collagen and avoiding unsightly keloid formation.

An active ingredient comprising the composition of the present invention is allantoin, which exerts a strong stimulation of cell proliferation and the reconstruction of the epidermis with high keratolytic activity dissolving the intercellular cement that holds the corneocytes together, by replacing dead cells for new and healthy ones. This favours the process of epithelisation, the basic key to the healing process, since this is capable of modifying cell proliferation of the basal cells. At the same time, allantoin has humectant activity, as a result of its ability to increase water retention in the cell matrix, a fundamental aspect for correct wound healing.

Another active substance comprised in the composition of the present invention is bisabolol, which has a calming and anti-free radical's effect, providing a slight anti-inflammatory and soothing action on the skin, when this is irritated.

Thus, the present invention discloses a pharmaceutical or cosmetic composition, comprising urokinase, allantoin and bisabolol and a dosage form of topical administration of said composition, involved in the processes of regeneration and healing of significant skin disorders in an integrated form, by using only one cosmetic preparation from the start of treatment, that ensures a favourable, continous and fast progressive evolution of significant skin disorders.

With the dosage form for topical administration, object of the present invention, an effect is obtained locally, with the systemic absorption of active ingredients being nil. Said dosage form comprises urokinase in combination with allantoin and bisabolol as active ingredients, to be applied in gel form. Additionally it comprises other active ingredients.

The dosage form, object of the present invention, maintains the physical-chemical and microbiological stability of the urokinase enzyme without the need for refrigeration at a temperature of less than 40 degrees centigrade.

In a preferred embodiment, the dosage form obtained in the present patent application in gel form comprises the urokinase enzyme in combination with allantoin and bisabolol and further comprises at least one active ingredient selected from active ingredients with regenerating function, such as hyaluronic acid, collagen, rosehip oil, boswellia serrata extract, antioxidants, enzymes and growth factors. This dosage form enables, with a single formulation, to address the entire process of regeneration and healing, including the phases of skin granulation, epithelialisation and remodelling, that constitutes a marked advantage compared with standard treatments, where each phase is approached individually with the active ingredients applied individually, leading to greater costs and treatment times.

Tests conducted with the pharmaceutical or cosmetic composition and the dosage form, object of the present invention, show a meaningful and effective acceleration in the process of healing the injured skin.

The application of the pharmaceutical or cosmetic composition and the dosage form, in gel form, object of the present invention, will be performed on the surface of the skin where the significant skin disorder is located, in the appropriate quantity, this being a volume of application of less than 5 ml, only once a day, for a period of 3-15 days. This application can be made directly on the skin through a light massage until fully absorbed or by performing a humid wound treatment using for this a an appropriate additional dressing.

The invention is better illustrated by the non limiting examples that follow.

### DESCRIPTION OF THE FIGURES

Figure 1 shows the performance of the PUSH scale score during the days of treatment with different groups of patients.
Figure 2 shows the average performance of the PUSH scale score during the first 72 hours of treatment with different groups of patients.

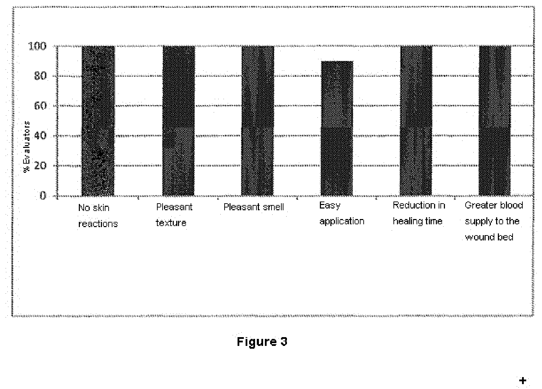

Figure 3 shows the results obtained by the evaluators in terms of different parameters of the pharmaceutical or cosmetic composition object of the present invention.
Figures 4.1, 4.2, 4.3 and 4.4 show the evolution of a wound in the first 72 hours, to which the pharmaceutical or cosmetic composition, object of the present invention, has been applied.

### DESCRIPTION OF PREFERRED EMBODIMENTS OF THE INVENTION

### Example 1

In a preferred embodiment, the pharmaceutical or cosmetic composition of the present invention comprises the following components:

| Components: | Concentration (% w/w) |
|---|---|
| Urokinase (1 IU/ml- 10 000 IU/ml) | 0.01 |
| Allantoin | 8 |
| Bisabolol | 1 |
| Isopropyl myristate | 25 |
| Glycerine | 15 |
| Xanthan gum | 2 |
| Preservatives (antimicrobial and antifungal) | 2 |
| pH regulators (acids and bases) | 5 |
| EDTA | 2 |
| Tocopherol | 1 |
| Water | q.s. 100 |

### Example 2

In order to evaluate the activity of the composition with regenerating and healing activity, the PUSH scale was used (for its acronym in English, Pressure Ulcer Scale for Healing), validated for monitoring the tracking of the process of wound healing. The measurement of this scale is intended to determine, with the highest possible reliability, the evolution of a pressure established ulcer, observing its development chronologically.

The PUSH scale evaluates the wound and assigns a score on the following aspects throughout the treatment:
- wound dimensions (length x width) from 0 to 10 points
- amount of exudate: 0 (nil) to 3 (abundant)
- tissue type: 0 (closed skin) to 4 (necrotic tissue)

A group of 10 volunteers (Case I - Case X) selected from different hospitals, with wounds in different anatomical locations, were treated with the composition described in Example 1, in gel form for topical application.

The PUSH scale was assessed at baseline level, in each case for 15 days; showing the values of greatest clinical relevance. During the 15 days of testing, the effectiveness of the treatment with the composition, object of the present invention, was confirmed, without any evidence of the decrease in the activity of the active ingredients.

At the same time, the PUSH scale was assessed in patients exclusively treated with urokinase (Urokinase Group), following the procedure of reconstituting, according to the manufacturer's instructions, vials of lyophilized powder of urokinase for intravenous use, soaking a gauze with this solution and applying the gauze on the injury to be treated. Under these conditions, the surplus of the urokinase solution must be kept under refrigeration a maximum of 72 hours, amongst other reasons because beyond this time the enzyme loses its activity, due mainly to bacterial contamination of the preparation.

Finally, using the PUSH scale, patients undergoing conventional treatment (Conventional Treatment Group), specifically with the usual antiseptics and hydrocolloid type dressings, were assessed.

Table 1 shows the PUSH values measured by days in each group of patients.

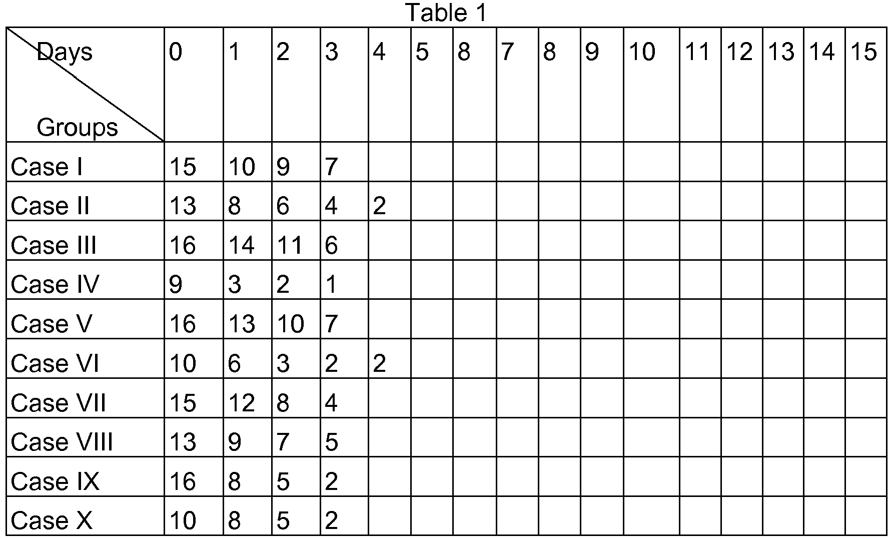

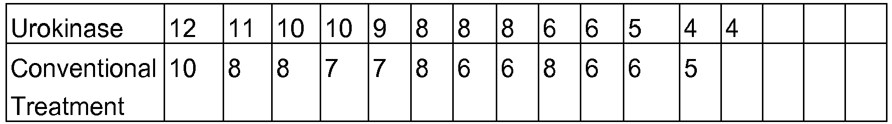

Table 1 shows that, during the first three days of treatment with the pharmaceutical or cosmetic composition, object of the present invention, in the cases in which a single daily application of the pharmaceutical or cosmetic composition, object of the present invention were made, there is an extraordinarily significant reduction of the values obtained on the PUSH scale.

On the other hand, in the case of the patients treated with the enzyme urokinase as an active ingredient (Urokinase group) and the patients undergoing conventional treatment (Conventional Treatment Group), the processes took much longer, surpassing even more than 2 weeks of treatment to achieve an acceptable clinical response, this response being reflected in the values obtained with the PUSH scale. Additionally, in these two groups, it was necessary to apply 3 treatments daily.

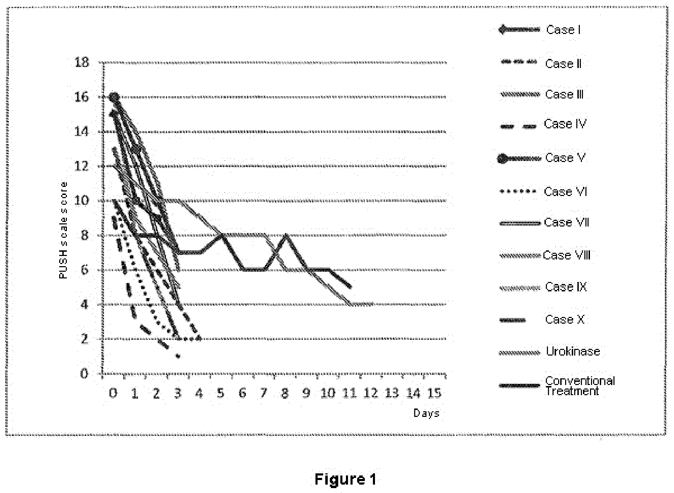

In any case it is also observed that if we compare those treatments where only the enzyme urokinase has been used with the conventional treatment, there are oscillations over time, demonstrating that the use of topical urokinase reduces the PUSH scale score basically by stimulating the formation of granulation tissue. These results are also shown in the graph in Figure 1. This graph demonstrates that during the first 72 hours of treatment and a single daily application with the composition object of the present invention, the healing process is accelerated compared with conventional treatment and the treatment with urokinase in dissolution of lyophilized powder, where many more days are necessary and even three applications per day are required.

### Example 3

In the following table (Table 2) the effectiveness and efficiency with respect to the treatment applied is demonstrated during the first three days. An average of the PUSH scale is made for each day of treatment and with a single application each day of all the cases treated with the composition, object of the present invention, described in Example 1. Similarly, on the same scale, a monitoring is made of a group of patients using only the composition excipients and the other group using only the regenerating agents. In all the groups and cases there was a single daily application.

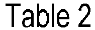

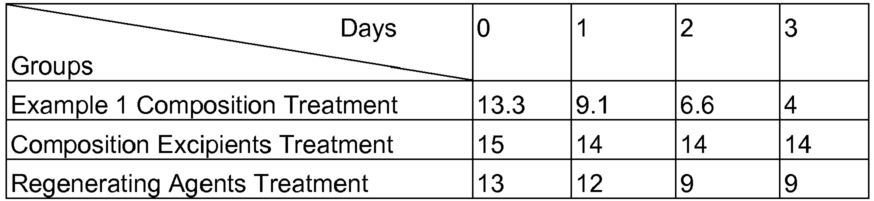

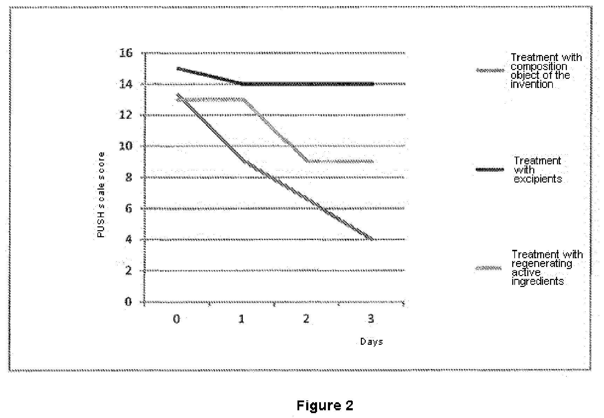

As shown in the previous table, in the group treated only with the excipients of the composition, object of the present invention, no significant reduction in the PUSH scale score is observed. In the group treated with regenerating ingredients, a discrete reduction in the PUSH scale score can be seen, basically determined by the reduction in the size of the wound. In the group treated with the composition, object of the present invention, an obvious reduction in the PUSH scale score during the first three days of treatment is observed, mainly by the reduction in the size of the wound and the type of exposed tissue. See Figure 2.

### Example 4

Other tests were made to assess the tolerability and safety of use of the pharmaceutical or cosmetic composition, object of the present invention, and the dosage form of topical administration also object of the present invention. The discomfort of employment, the emergence of adverse skin reactions, irritability in the area of application and pruritus, all on the basis of an adapted questionnaire, were evaluated.

None of the volunteer participants in the tests presented any undesirable skin reactions, such as a rash, irritation, or itching associated with the application of the pharmaceutical or cosmetic composition during the treatment, which in some cases lasted more than 15 days.

All the volunteer participants in the tests considered the texture and smell of the applied product pleasant, also considering the application quick and easy.

The results obtained showed that the pharmaceutical or cosmetic composition of the present invention, applied in gel form, accelerates the healing process with respect to other treatments available in the market.

Also, the results showed that the pharmaceutical or cosmetic composition used increased the supply of blood to the wound bed, corroborated by the visual evaluation of new blood vessels and the emergence of granulation tissue, essential for the correct healing of any significant skin disorder.

The above results are shown in Table 3.

### Example 5

In a preferred embodiment, the pharmaceutical or cosmetic composition object of the present invention is prepared in gel form for topical administration.

The prepared gel includes pharmaceutical or cosmetically acceptable compounds selected from the following list as excipients:
- Viscosity controlling agents between 0.5 and 8% weight/weight, such as cellulose ethers, pectins, alginates, gum arabic, guar gum, xanthan gum or agar agar, among other regulatory agents of viscosity.
- Humectants between 1 and 25% weight/weight, such as glycerine, urea, sorbitol, propylene glycol, other glycolic solvents, nitrogen compounds, fatty alcohols, alphahydroxy acids in low concentrations or sugar polymers, among others.
- Acids, pH bases or regulatory buffer solutions, between 0.1 and 10% weight/weight.
- Emollients between 1 and 25% weight/weight, such as vegetable oils and their esters, mineral oils, fatty alcohols and their derivatives, phospholipids, silicones or ethoxylated glyceride, among others.
- Chelating agents between 0.05 and 5% weight/weight, such as EDTA salts (ethylenediaminetetraacetate), iminodisuccinate salts and acid forms thereof or polyaspartic acid and salts thereof, among others.
- Preservatives such as antimicrobial and antifungal agents, organic acids and salts thereof, alcohols, phenol derivatives, formaldehyde donors or halogenated derivatives, among others, between 0.01% and 8% weight/weight.
- Antioxidants between 0.01 and 3% weight/weight, from vegetables or fruits or synthetics such as anthocyanins, flavonoids, tocopherols and derivatives thereof, ascorbic acid and derivatives thereof, beta carotene, albumin, uric acid, bilirubin, methionine sulfoxide reductase, superoxide dismutase, glutathione peroxidase, binding proteins to metals, butyhydroxytoluene or butyhydroxyanisole or DNA repair enzymes, among others.
- Water, q.s.

The gel that carries the active ingredients is a semi-solid polymer of transparent white colour, which has a pleasant smell and does not stain skin or fabrics. pH regulators, preservatives and stabilisers provide activity and stability needed for the active ingredients to exercise their activity, which is demonstrated through the reported clinical cases.

The gel has rheological properties suitable for an appropriate extensibility and adaptability to the surface to be treated, which remains attached to the surface forming a thin film in contact with said surface. It is also easily washable.

The activity and physical-chemical and microbiological stability of the gel are maintained without refrigeration for a period of at least 18 months in an unopened container of gel and a period of 3 months after opening the package container of gel, always at temperatures below 40°C. All the excipients guarantee the activity and stability of the active ingredients, after successfully passing the tests of stability under accelerated conditions (data not shown). Viscosity controlling agents mainly guarantee the physical-chemical stability of the gel creating a protective micro-environment and a support matrix that immobilises the urokinase enzyme, avoiding its autolysis and stabilising it in this medium. The microbiological stability of the gel and urokinase is determined by preservatives, since these have successfully passed the preservative's effectiveness tests.

The tests made showed that said dosage form in gel maintains the effectiveness and stability of the enzyme for long periods of time, at least 18 months, in using the same preparation of pharmaceutical or cosmetic composition during the 15 days of the testing, this preparation having been made 18 months in advance, and obtaining the same results as with the preparations of pharmaceutical or cosmetic composition, object of the present invention, prepared every 72 hours and maintained at 4 °C, the latter being the method that is applied when vials of lyophilized powder of the urokinase enzyme is reconstituted for intravenous use with the solvent, according to the manufacturer's instructions, where the urokinase is stable within a maximum period of 72 hours. (Data not shown).

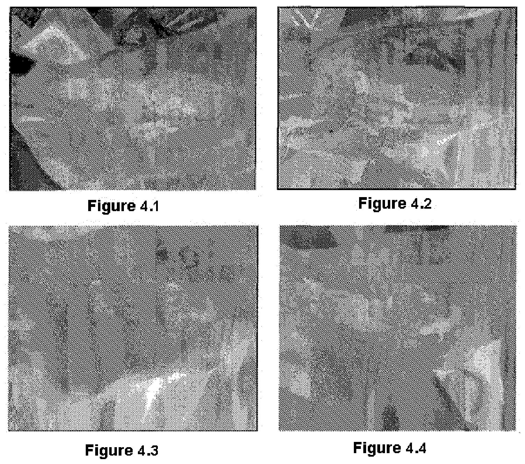

The gel is applied in a volume of less than 5 ml in each daily application. An example of the results obtained with the gel for topical administration of pharmaceutical or cosmetic composition, object of the present invention, is shown in Figures 4.1 to 4.4, where the favourable evolution in regeneration and healing of a wound in the first 72 hours of treatment can be seen.

In other pharmaceutical forms, the pharmaceutical or cosmetic composition, object of the present invention, may be presented in the form of lotions, creams, milks, suspensions, liquid crystals or foams. It could also have different presentation formats, such as multidose or single dose packages, impregnated carriers, sticks, and other forms known to the expert in the art of topical applications.

## Claims

1. A pharmaceutical or cosmetic composition **characterised in that** it comprises urokinase in combination with allantoin and bisabolol as active ingredients.

2. The pharmaceutical or cosmetic composition, according to Claim 1, **characterised in that** the urokinase is in a concentration between 1 IU/ml and 10 000 IU/ml, the allantoin between 5-8 % w/w and the bisabolol between 0.01-7 % p/p.

3. The pharmaceutical or cosmetic composition, according to Claim 2, **characterised in that** it further comprises, at least, one active ingredient selected from hyaluronic acid, collagen, rosehip oil, boswellia serrata extract, antioxidants, enzymes and growth factors.

4. A pharmaceutical or cosmetic composition according to claim 1 for use in the treatment of skin disorders selected from the group consisting of:
a. superficial erosions;
b. minor irritations;
c. ulcer type injuries of venous or arterial origin;
d. pressure ulcers;
e. diabetic foot;
f. traumatic wounds; and
g. wounds of surgical origin.

5. The pharmaceutical or cosmetic composition for use according to claim 4, wherein said composition is to be administered topically in gel form.

6. The pharmaceutical or cosmetic composition for use according to claim 4, wherein said composition to be administered in a single daily dose for a period of 3 to 15 days.

7. The pharmaceutical or cosmetic composition for use according to claim 4, wherein the composition is in a topical dosage form.

8. The pharmaceutical or cosmetic composition for use according to claim 4, wherein the administration volume is below 5 ml per application.

9. A topical dosage form comprising the composition according to Claim 1.

10. The topical dosage form, according to Claim 9, **characterised in that** it further comprises at least one active ingredient selected from hyaluronic acid, collagen, rosehip oil, boswellia serrata extract, antioxidants, enzymes and growth factors.

11. The topical dosage form, according to any one of Claims 9 to 10, **characterised in that** the gel has between 0.5 and 8% viscosity regulating agents, between 1 to 25% humectants, between 0.1 to 10% pH regulators, between 1 and 25% emollients, between 0.05 to 5% chelating agents, between 0.01% and 8% preservatives and between 0.1 to 3% antioxidants, with concentrations being % weight/ weight, so that the individual values of the different components of the composition are such that the total composition never exceeds 100%.

## Patentansprüche

1. Pharmazeutische oder kosmetische Zusammensetzung **dadurch gekennzeichnet, dass** sie Urokinase in Kombination mit Allantoin und Bisabolol als aktive Inhaltsstoffe enthält.

2. Pharmazeutische oder kosmetische Zusammensetzung nach Anspruch 1 **dadurch gekennzeichnet, dass** die Urokinase in einer Konzentration zwischen 1IU/ml und 10000 IU/ml, Allantoin zwischen 5-8 % w/w und Bisabolol zwischen 0,01-7 % p/p vorliegt.

3. Pharmazeutische oder kosmetische Zusammensetzung nach Anspruch 2 **dadurch gekennzeichnet, dass** sie weiterhin zumindest einen aktiven Inhaltsstoff aufweist ausgewählt aus Hyaluronsäure, Kollagen, Hagebuttenöl, Boswellia serrata Extrakt, Antioxidantien, Enzyme und Wachstumsfaktoren.

4. Pharmazeutische oder kosmetische Zusammensetzung nach Anspruch 1 zur Verwendung bei der Behandlung von Hautstörungen ausgewählt aus der Gruppe bestehend aus:
a. Oberflächliche Erosion;
b. Kleine Irritationen;
c. Verletzungen vom Ulcus-Typ venösen oder arteriellen Ursprungs;
d. Druck Ulcus;
e. Diabetische Füße;
f. Traumatische Wunden; und
g. Wunden chirurgischen Ursprungs.

5. Pharmazeutische oder kosmetische Zusammensetzung zur Verwendung nach Anspruch 4, wobei die Zusammensetzung topisch, in Gel-form zu verabreichen ist.

6. Pharmazeutische oder kosmetische Zusammensetzung zur Verwendung nach Anspruch 4, wobei die Zusammensetzung in einer einzigen täglichen Dosis für einen Zeitraum von 3 bis 15 Tagen zu verabreichen ist.

7. Pharmazeutische oder kosmetische Zusammensetzung zur Verwendung nach Anspruch 4, wobei die Zusammensetzung in einer topischen Dosierungsform vorliegt.

8. Pharmazeutische oder kosmetische Zusammensetzung zur Verwendung nach Anspruch 4, wobei das Verabreichungsvolumen unterhalb von 5 ml pro Anwendung liegt.

9. Topische Dosierungsform umfassend die Zusammensetzung nach Anspruch 1.

10. Topische Dosierungsform nach Anspruch 9 **dadurch gekennzeichnet, dass** sie weiterhin zumindest einen aktiven Inhaltsstoff aufweist ausgewählt aus Hyaluronsäure, Kollagen, Hagebuttenöl, Boswellia serrata Extrakt, Antioxidantien, Enzyme und Wachstumsfaktoren.

11. Topische Dosierungsform nach einem der Ansprüche 9 bis 10 **dadurch gekennzeichnet, dass** das Gel aufweist Viskosität regulierenden Agenzien zwischen 0,5 und 8 %, zwischen 1 und 25 % Befeuchtungsmittel, zwischen 0,1 und 10 % pH Regulatoren, zwischen 1 und 25% Weichmacher, zwischen 0,05 und 5 % Chelatoren, zwischen 0,01% und 8% Konservierungsmittel und zwischen 0,1 und 3 % Antioxidantien mit Konzentrationen in % Gewicht/Gewicht, so dass die individuellen Werte der verschiedenen Komponenten der Zusammensetzung so gewählt sind, dass die gesamte Zusammensetzung nie 100% überschreitet.

## Revendications

1. Composition pharmaceutique ou cosmétique **caractérisée en ce qu'**elle comprend, en tant que principes actifs, de l'urokinase en combinaison avec de l'allantoïne et du bisabolol.

2. Composition pharmaceutique ou cosmétique selon la revendication 1, **caractérisée en ce que** l'urokinase est à une concentration comprise entre 1 UI/ml et 10 000 UI/ml, l'allantoïne entre 5 et 8 % en poids et le bisabolol entre 0,01 et 7 % en poids.

3. Composition pharmaceutique ou cosmétique selon la revendication 2, **caractérisée en ce qu'**elle comprend en outre au moins un principe actif choisi parmi l'acide hyaluronique, le collagène, l'essence de cynorhodon, l'extrait de Boswellia serrata, les antioxydants, les enzymes et les facteurs de croissance.

4. Composition pharmaceutique ou cosmétique selon la revendication 1 pour une utilisation dans le traitement de troubles cutanés choisis dans le groupe constitué par :
a. les érosions superficielles ;
b. les irritations mineures ;
c. les lésions de type ulcère d'origine veineuse ou artérielle ;
d. les escarres ;
e. le pied diabétique ;
f. les plaies traumatiques ; et
g. les plaies d'origine chirurgicale.

5. Composition pharmaceutique ou cosmétique pour une utilisation selon la revendication 4, laquelle composition est destinée à être administrée par voie topique sous la forme d'un gel.

6. Composition pharmaceutique ou cosmétique pour une utilisation selon la revendication 4, laquelle composition est destinée à être administrée en une seule dose quotidienne sur une période de 3 à 15 jours.

7. Composition pharmaceutique ou cosmétique pour une utilisation selon la revendication 4, laquelle composition est sous une forme posologique à usage topique.

8. Composition pharmaceutique ou cosmétique pour une utilisation selon la revendication 4, dans laquelle le volume d'administration est inférieur à 5 ml par application.

9. Forme posologique à usage topique comprenant la composition selon la revendication 1.

10. Forme posologique à usage topique selon la revendication 9, **caractérisée en ce qu'**elle comprend en outre au moins un principe actif choisi parmi l'acide hyaluronique, le collagène, l'essence de cynorhodon, l'extrait de Boswellia serrata, les antioxydants, les enzymes et les facteurs de croissance.

11. Forme posologique à usage topique selon l'une quelconque des revendications 9 et 10, **caractérisée en ce que** le gel a entre 0,5 et 8 % d'agents régulateurs de viscosité, entre 1 et 25 % d'humectants, entre 0,1 et 10 % de régulateurs de pH, entre 1 et 25 % d'émollients, entre 0,05 et 5 % d'agents chélatants, entre 0,01 % et 8 % de conservateurs et entre 0,1 et 3 % d'antioxydants, les concentrations étant en pourcentages en poids, les valeurs individuelles des différents composants de la composition étant telles que la composition totale ne dépasse jamais 100 %.
